# EUROPEAN PATENT SPECIFICATION

(11) **EP 1 196 625 B1**
(45) Date of publication and mention of the grant of the patent: **09.08.2006**
(21) Application number: 01925277.4
(22) Date of filing: 17.04.2001
(51) Int. Cl.: C12Q 1/04, C12R 1/19, C12R 1/22, C12R 1/37, C12R 1/385, C12R 1/42

(54) **COMPOSITION AND METHOD FOR DETECTING AND EARLY AND DIFFERENTIATED COUNTING OF GRAM-NEGATIVE MICROORGANISMS**
ZUSAMMENSETZUNG UND VERFAHREN ZUM NACHWEIS UND ZUM FRÜHEN UND DIFFERENZIERTEN ZÄHLEN VON GRAMNEGATIVEN MIKROORGANISMEN
COMPOSITION ET PROCEDE DESTINES A LA DETECTION, AU COMPTE ET A LA DIFFERENTIATION DE MICRO-ORGANISMES GRAM NEGATIFS

(30) Priority: 18.04.2000 CU 832000
(43) Date of publication of application: 17.04.2002
(73) Proprietor: CENTRO NACIONAL DE BIOPREPARADOS, Bejucal, Provincia La Habana 6048 (CU)
(72) Inventor: QUESADA MUNIZ, Vivian de Jesus, Quivicán, Provincia la Habana (CU); RODRIGUEZ MARTINEZ, Claudio, Provincia la Habana (CU)
(74) Representative: Prins, Adrianus Willem
(86) International application number: PCT/CU2001/000002
(87) International publication number: WO 2001/081615

(56) References cited:
- WO-A-96/30543
- ES-A- 2 079 319
- US-A- 5 194 374

## Description

### Technical sector

The present invention is related with the Microbiology field and particularly with a composition and a method for early detection, identification, differentiation and count of microscopic organisms, concretely Gram-negative microorganisms.

### Prior Art

The recuperation, identification and count of Gram-negative microorganisms, such as *Salmonella, E. coli* and coliforms group, are of a great interest in the clinical diagnosis and in the sanitary quality control of waters, foods and environmental samples.

For the identification and count of the microscopic Gram-negative organisms, exists a range of culture media with formulations that could be considered "traditional", many of them developed from the past century. Within these media could be marked S.S. Agar, S.S. Agar (modified), XLD Medium, Hektoen Enteric Agar, Kristensen Agar and Brilliant Green Agar, used for the identification of *Salmonella* (Soria Melquizo, F. Difco Handbook. Tenth edition. 1984; MERCK Handbook of Culture Media. 1990; OXOID Handbook of Culture Media. 1995)

In general, these media have an inconvenient: they are inhibitory to a great number of enteric bacteria, even *Salmonella's* growth is observed lightly inhibited due to the use of inhibitors that overcome the growth promoting properties of the nutrients that media contain.

Some of the culture media previously referred, base the *Salmonella* identification on the H₂S production, but this reaction is also characteristic for other Gram-negative organisms, such as *Proteus* and *Citrobacter.*

Other media have been thoroughly marketed for the identification of *E*. *coli and* coliform bacteria count in several biological samples, such as Endo Agar, Eosin Methylene Blue Agar, MacConkey Agar, Violet Red Bile Agar, among others (Soria Melquizo, F. Difco Handbook. Tenth edition. 1984; MERCK Handbook of Culture Media. 1990; OXOID Handbook of Culture Media. 1995).

These media, in general, contain inhibitors of Gram-positive organisms, which not being in adequate proportion in relation to the nutrients; frequently reduce the growth of target microorganism. On the other hand, in these media is not possible to identify nor *Salmonella* neither the late lactose fermenting organisms.

Diagnostic specificity and/or sensibility of these media generally are not so high, because they base the identification of such organisms on carbohydrate fermenting biochemical reactions that are common for several target species or genera, as it is the case of lactose degradation by the coliform group (since not all are able to ferment this substrate).

In 1998, Helena Tuompo et al patented a method and a culture medium for the identification of *Salmonella* (Patent No. US 5, 786, 167), based on the ability of this microorganism of metabolize melibiose, mannita and sorbita, as well as on the inclusion of a chromogenic substrate, in order to detect β-galactosidase activity.

This medium has the limitation that it does not allow to identify *Proteus* species- an enteric bacteria of sanitary interest, since this organism grows as colorless colonies.

Another inconvenient that presents this medium is that it does not allow the count of enteric bacteria, and *Salmonella* could be confused with mannita fermenting *Shigella* as *S. sonnei* and *S. flexneri*.

That same year Alain Rambach patented a chromogenic-fluorogenic culture medium to detect *E. coli* and the method for its use (Patent No. US 5, 846, 761).

The invention consisted in the use of a substrate derived from indolyl-glucuronic acid or it's salts, and a non-chromogenic substrate derived from alkyl, -alkenyl or - aryl of the glucuronic acid or their salts.

This medium is specific for *E.coli* and it does not allow the identification and/or count of *Salmonella* and other microscopic Gram-negative organisms. On the other hand it requires the use of an ultraviolet light lamp, which makes difficult the count.

In the patent No. US 5, 723, 308, Mach et al describe a medium for the rapid count of coliform bacteria. The medium includes a mixture of gelatin peptone and yeast extract, lactose or glucose, sodium chloride, bile salts, arabic gum and pH indicators such as phenol red and sulfonftalein, in quantities from 0,16 to 5 g/ L, in order to identify the bacteria. These high concentrations of indicators are, at the same time, inhibitory and cause the poor or no growth of target microorganisms.

The principle of the sugar fermentation, specifically lactose fermentation by coliform organisms, is still being used in this medium with the disadvantage of not allowing the detection of the late fermenting organisms such as, for example *Citrobacter.* Other enteric bacteria are not identified and could be also affected by the high concentration of indicators.

In 1993 Christopher Tate et al patented a very selective medium for *Salmonella* (Patent No. US 5, 208, 150), with the inclusion of Tergitol RTM in a base with xylose-lysine, where sulfapyridine is also included for the inhibition of the *Citrobacter* growth. As for the majority of the media that have been analyzed and that are currently in the market for the *Salmonella* identification, the problem is solved with the inhibition of the flora, which could be confused with *Salmonella,* mainly *Proteus* and *Citrobacter.* These enteric bacteria, specifically *Citrobacter* is considered a coliform, therefore the count of these organisms seems to be affected in this medium. The use of an antibiotic in the medium, as novobiocin, becomes an inconvenient for the preparation of the medium. Additional cares are needed for its sterilization and conservation.

Alain Rambach patented a medium for the isolation and identification of *Salmonella* (Patent No. US 5, 194, 374), which could be considered the nearest prototype to this invention. It consists in the identification of this organism by means of the splitting reaction of 1, 2-propanodiol in presence of a pH indicator, which is adsorbed in a powdered material, with a particle size of 100 microns. Such material could be silica, silica gel or cellulose. Besides other components, in this medium is included a substrate for β-galactosidase, IPTG and deoxycholates.

The medium previously referred shows the following disadvantages:
- Not all *Salmonellas* "non typhi" give red color, because according to the reports of Monget-Daniel (Patent No. US 5, 434, 056), one strain of each one of the *Salmonella enteritidis, S. gallinarum, S. pullorum and S.paratyphi A* assayed by him, did not developed any color, and one strain of S. *Arizonae* developed a blue color.
- One strain of *Klebsiella pneumoniae* showed a blue color in several tests carried out in the laboratory, when the expected reaction should be violet, taking in account that this organism metabolizes the propane-1,2-diol, it is β-galactosidase positive and the majority of the strains of this organism reveals a blue color after 24 hours.
- In experiments carried out during the development of this invention, the results of Monget were corroborated, finding some strains of *Pseudomonas* with red color, characteristic also for *Salmonella.*
- Unexpected blue color was detected also for strains of *Proteus vulgaris.*
- The medium is not designed for the organisms count, due fundamentally to the composition of nutrients, which do not include the necessary amount of each nutrient in order to overcome the inhibitory power of the deoxycholates included in the formulation. Likewise, the total relationship between nutrients and inhibitors is not enough and does not allow an adequate development of the Gram-negative organisms, and thus finally does not guarantee a sure count of such organisms.
- The previous deficiency shows its negative influence when the Rambach medium is employed for the identification of *Salmonella,* because of the necessity to carry out a preceding incubation of the sample in a pre-enrichment or enrichment medium, before being inoculated in the Rambach Agar plate. Generally, the period of this enrichment stage extends for 18-24 hours in Selenite Broth, Tetrationate or Rappaport Vassiliadis media.
- In different tests carried out by the applicant of the present invention, strains of *Enterobacter aerogenes* (ATCC 13048) and *Klebsiella pneumoniae* (ATCC 13883), appeared very inhibited in the medium after 18 to 24 hours of incubation at 37 °C. *Proteus vulgaris* (ATCC 13315) and *Proteus mirabilis* (ATCC 7002) did not grow in the same experiment. In another two series of tests, *Enterobacter aerogenes* ATCC 13048 did not grow in the medium, in comparison with experimental media.
- Another disadvantage is the quantity of powdered silica gel that is included in the described medium, it makes difficult its homogeneous distribution and silica easily flocculates, provoking, in many cases, the appearance of a non-adequate consistency of the medium and a troublesome manipulation.
- The high adsorption capacity of the silica does not allow the diffusion of the adsorbed components in the medium.
- The low growth promotion capacity influences even in the growth of *Salmonella,* because in previously carried out experiences, a strain of *Salmonella typhi*, resulted inhibited in the medium.
- The relationship between inhibitors, nutrients and chromogenic substances is not the most appropriate, since it is necessary to include activators of the enzymes (IPTG) in the formula, in order to metabolize the substrates and to develop the reaction in less time.

### Disclosure of the Invention

The aim of this invention consists on providing a composition and a method for early detection and differential count of microscopic Gram-negative organisms, allowing, specifically, to identify and carry out a sure and simultaneous detection and/or count of *Salmonella, E. coli*, coliforms and other Gram-negative bacteria as: *Pseudomonas, Citrobacter* and *Klebsiella,* in early cultivation periods and with a high analytic sensibility.

The novelty of the solution consists on providing mixtures of highly nutritious substances of protein origin, with a high content of total nitrogen- between 10 and 33 %. The content of these mixtures in the formulation is in a relationship from 2:1 to 24:1 to the total content of the inhibitory substances for organisms which have to be inhibited, and more specifically Gram-positives.

On the other hand, in the composition, mixtures of organic and inorganic substances are included, which allows to make a differential count of the target microscopic organisms, being this mixture in an adequate relationship from 0,5: 1 to 2: 1 to the mixture of nutritive substances of protein origin, mentioned before.

The substances of protein origin are selected from several types of compounds, among of them could be mentioned:
- Dehydrated pancreatic or papainic beef heart hydrolysates, with a total nitrogen content from 10 to 15 %, which are in the mixture of substances of protein origin in concentrations from 25 to 75 %.
- Dehydrated enzymatic milk protein hydrolysate with a total nitrogen content from 11 to 20 %, which could be in the mixture in concentrations up to 15 % of said mixture of protein substances.
- Enzymatic microbial autolysates or hydrolysates, with a total nitrogen content from 8 to 15 %, and which are in said mixture in concentrations from 15 to 25 %.
- Mixtures of egg yolk proteins, with total nitrogen content from 15 to 33 %, which could be in the mixture in concentrations up to 45 %.

The composition described in this invention includes small quantities of inhibitors of the growth of Gram-positive microscopic organism, such as cholic and deoxycholic acids as well as bile salts, which are in the composition in a relation between 0.5:1 to 2:1 in respect to the mixture of substances of protein origin, it means in amounts from 0,8 to 4 g/ L.

On the other hand, a novel aspect of the invention consists on the jointly use of compounds previously mentioned, with other compounds, which allow the selective differentiation of target organisms, being the last group of compounds selected from bivalent metal's oxides and siliceous compounds, such as 3MgO x 4SiO₂ x H₂O, SiO₂ xH₂O and SiO₂. Said substances are in quantities from 2 to 20 g/L (concentrations from 6 to 32% respecting to the total mass of the mixture) together with phenol red or neutral red in quantities from 0,01 to 0,06 g/L (concentrations from 0.03 to 0.18% of the total mass), in presence of alcohols, and said alcohols could be metabolized by the enzymes of some of the target organisms, preferably C₃H₈O₂, in quantities from 10 to 14 mUL and in the presence of chromogenic compounds. Said chromogenic compounds could be metabolized by other microscopic organisms different from those that could metabolize said alcohols, and which, when being metabolized, give to the colonies several colors, different from those colors, characteristic of the organisms which are able to metabolize said alcohols, preferably X-gal in quantities from 0,05 to 0,1 g/L (concentrations from 0.15 to 0.3% of the total mass of the mixture).

As another novel element in the formulation for this type of medium for the proposed finality, the combination of sodium and magnesium salts, such as magnesium chloride and sodium carbonate, in quantities from 0,01 to 10 g/L was included (concentrations from 0.03% to 32% of the total mass of the mixture). Creatinine and low molecular weight nitrogen compounds could be included in quantities up to 1 g/L (up to 3% of the mixture), sulfured amino acids, such as cystine and cysteine in quantities up to 0,4 g/ L could be also included (concentrations up to 1.25% of the total mass of the mixture).

In the composition, gelling agents are included, preferably agar, with a hardness between 400 and 700 g/ cm², in quantities from 13 to 20 g/ L (from 40 to 63% of the total mass of the mixture).

The composition is reconstituted in water in order to be used, in quantities from 30 to 32 g/ L. For this purpose the alcohol is added to the water, shaking until the total distribution. The rest of the ingredients are added to the liquid mixture, shaking and heating to temperature of 100 °C for 1 to 3 minutes and decreasing the temperature to 45-50 °C. The new composition has a pH value from 6,8 to 7,4 (after boiling for 1-15 minutes and cooling until 20-25 °C).

The composition is employed incubating it in a gel form at temperature from 30 to 45 °C, for at least 12 hours.

In this prepared and restored in water composition, was possible to provide a new method for the early detection and differential count of Gram-negative microscopic organisms such as *Salmonella, E.coli* and other coliform organisms. *E. coli* and coliforms will show a blue-greenish color of the colony on an orange bottom of the medium. *Salmonella* (non *typhi)* can be detected by a red color on the center of the colonies on a rosy bottom. *Salmonella typhi* and *Proteus vulgaris* could be detected by the transparency of the colonies. *Citrobacter* and *Klebsiella* are differentiated by the violet color of the colony on the rosy or orange bottom of the medium. *Pseudomonas aeruginosa* could be detected by the clear orange color with darker center of the colony, and later becomes greenish after 24 h, and by the production of a yellow-greenish fluorescence under ultraviolet light.

The advantages of the new invention reside in that:
- For first time is provided a composition that allows the early detection and differentiated enumeration of several species and genera of microscopic Gram-negative organisms, among them *Salmonella typhi, Salmonella non-typhi, Escherichia coli, Proteus, Citrobacter* and *Pseudomonas.*
- The balance between the nutritive bases, with adequate total nitrogen content, the relationship between them, and with the inhibitors, facilitates that the target organisms grow without inhibition, especially *Salmonella,* and on the other hand, guarantees that Gram-positive organisms are totally inhibited.
- The diagnostic specificity for the target organisms in the composition is very high, because in all experiences carried out resulted 100 % for all the organisms.
- In an unexpected manner, the isolation and identification of *Salmonella* has been facilitated in only 12 hours, without necessity of the previous pre-enrichment or enrichment of the sample.
- The analytic sensibility is very high, even before 24 hours, for all target organisms.
- With the use of this composition is possible the early identification and enumeration of lactose late fermenting organisms that could be enumerated and identified as coliforms such as *Citrobacter* and *Serratia.*
- It is of great interest that in an unexpected manner *Proteus*, which is an organisms of sanitary interest and which is an enteric bacterium but not a coliform, and *Salmonella typhi*, another Gram negative bacterium, grow in the medium and are identified as colorless colonies, by which, their presence do not interfere in the count of the coliforms, and in this way, they are available for a further identification.
- Species of *Salmonella* have been enumerated in a differentiated form from the mannitol fermenting *Shigella,* such as *Shigella flexneri* and *Shigella sonnei.*
- Due to the balance achieved in the proposed formulation between inhibitors, nutritive bases and other growth promoting substances, and their relationship with the substrates (that allow the differentiated identification from the target organisms) is possible to use low concentrations of the indicators or revealer of the reaction (0,01 to 0,06 g/ L), by which the promoting or nutritive qualities of the product are intensified.
- Low concentrations of indicators are enough for identifying the target organisms, with the saving of these substances, difficult of being obtained by synthesis.
- The substances that conform the composition are thermo-stable and none complicates the preparation of the final medium. They facilitate the preparation of the diagnostic device, without the necessity of sterilizing it.
- The reactions that allow the identification of the target organisms in this composition are clear and common for the strains of each genera or species to identify. None "atypical" responses have been observed, neither for the *Salmonella* nor for the rest of the enteric bacteria, including the coliform group.
- Surprisingly, when using this composition, strains of *Klebsiella* gave a different response to the rest of enteric bacteria, except to *Citrobacter.* This constitutes a new diagnostic possibility because it does allow the presumptive count of these two germs whenever it is required.
- With this invention, a considerable reduction of the total time of the assay is achieved. In the case of E. *coli,* in certain applications, in less than 12 hours could be achieved as result.
- The product does not contain substances difficult to distribute or to dissolve; neither contains incompatible components, by which its preparation is simple.
- The conjunction of several sources of especially selected proteins and proteinaceous substances derived from their hydrolysis, with a nitrogen content from 8 to 33 %, provides enough nutrients for the development of all target genus and species without inhibition in a short period of time. The presence of other components, such as vitamins and microelements favors this balance.
- The invention provides a relationship between the components of organic and inorganic nature and proteinaceuos substances from 0,5:1 to 2:1, which facilitates the differentiation of the target organisms. This relationship allows the growth of the organisms easily inhibited by the group of cholic and deoxycholic acid compounds, and which are difficult to be differentiated by the traditional methods of sugars degradation.
- The addition of bivalent metals oxides and siliceous in quantities from 2 to 20 g/L, besides of activating the microbial metabolism, allows conforming a solid structure, which in conjunction with agar of a selected hardness (400-700 g/cm²) allow the formation of characteristic colonies. The color of the colonies does not diffuse in the medium, by which finally, facilitates the simultaneous differentiation by colors of a wide range of microscopic organisms.
- The mixture of egg proteins, besides of contributing with nutrients, makes possible, jointly with mentioned in the previous paragraph substances, the appearance of a contrast zone in all the volume of the medium, and this facilitates the detection of several colors and tonalities of the colonies.
- The combination of alcohols degradable by the enzymes of at least one of the organisms to identify, in the established quantities, with the pH indicators and X-gal, facilitates that, at high dilutions of the sample and at few hours from the beginning of the cultivation, (less than 12 hours for *E. coli* and about 12 hours for *Salmonella* and coliforms bacteria), the reactions could be observed and by this mean the right and sure identification is achieved, facilitating the high sensibility and specificity of the diagnostic device.
- The presence of sodium and magnesium salts in predicted quantities, allows to accelerate the enzymatic reactions and, in combination with other compounds such as creatinine and other sulfured amino acids, also offers additional nutrients for target organisms that are susceptible of being inhibited by cholic and deoxycholic acid compounds.
- The quantities in that the composition is restored in water, as well as the preparation method and incubation of the samples, provide an easy, simple and sure procedure for laboratory workers. It offers a superior reliability to the diagnostic procedure.
- The possibility that offers the new composition, for the identification of the microscopic organisms only through their chromatic peculiar characteristics, allows the reading and identification of the results by personnel with a minimum of preparation.

### Detailed description of the invention

To prepare the composition described in this invention, the components are prepared according to their physical state: solid or liquid. The preparation of the solid components or powders is described next.

The substances of protein origin selected within the group of pancreatic or papainic beef heart hydrolysate, with a total nitrogen content between 9-14 % and in a quantity from 25 to 77 % concerning the mass of the mixture of said substances of protein origin; enzymatic hydrolysate of milk proteins with a total nitrogen content from 9 to 20 %, and in a quantity from up to 15 % concerning the mass of said mixture, as well as the egg proteins, with a total nitrogen content from 5 to 7 % and in a quantity up to 45 % concerning the mass of the mixture, are sifted or processed in order to achieve the uniformity of the particles size.

These protein origin substances are in a relationship from 2: 1 to 24: 1 in relation to the content of inhibitors, selected within the group of the cholic and deoxycholic acid, bile salts and sodium deoxycholate. They are sifted, being ready to be mixed and homogenized with the rest of organic and inorganic components that are added in a relationship from 0,5: 1 to 2: 1, to the mass of the mixture of protein origin substances.

The pre-mixture is carried out combining bivalent metals oxides and/ or siliceous compounds (3MgO x 4SiO₂ x H₂O; SiO₂ x H₂O), which have been previously dried and taken in quantities between 6 and 32 % in relation to the mass of the final mixture; pH indicators, preferably phenol red or neutral red in quantities from 0,03 to 0,18 % in relation to the total mixture; chromogenic compounds that could be metabolized by the action of at least one organism, preferably X-gal in quantities from 0,15 to 0,3 %, concerning the total mass of the mixture. The pre-mixture of these compounds could be grinded and sifted before their homogenization, in a manner that weighing the sum of the quantity of each component in the formulation; an adequate distribution of each one is achieved.

Sodium and magnesium salts (sodium carbonate and magnesium chloride) previously are dried, ground and sifted before adding them to the final mixture in quantities of 0,03 to 32 %.

The nitrogen compound of low molecular weigh, specifically creatinine, (up to 3 % concerning the mass of the final mixture) and the sulfured amino acids preferably cystine and cysteine (up to 1,25% concerning the final mass of the mixture) are sifted before adding them to the total mixture.

The gelling agent, preferably agar, of hardness between 400-700 g/ cm², which is used in quantities between 40 - 67 % concerning the final mass of the mixture, is dried off and sifted before adding it to the final mixture.

All components mentioned before, as well as the pre-mixture, are poured together in a homogenizer and mixed until achieving a uniform content with a pH from 6,8 to 7,4. This mixture is poured into flasks and tightly closed, protected from the light, maintaining them at room temperature.

The liquid components of the composition, that could be the polyalcohols, specifically C₃H₈O₂, and the egg proteins without dehydration, as well as the distilled or deionized water, are mixed thoroughly until achieving a homogeneous solution.

The powder is added in quantities from 30 to 32 g/L to the liquid mixture, by this way the composition is reconstituted and the suspension of the composition is ready for use.

The composition could be prepared at laboratory scale, weighing the components in separate form within a container and maintaining all the before mentioned proportions. The liquid mixture is added subsequently on the powder mixture. The suspension is agitated and is allowed to swell for 10 minutes before bringing it to boil for a period of 1 to 3 minutes at least. The suspension is cooled down until 45 - 50 °C and distributed in the final assay container. The composition is let to solidify at temperature between 25-30 °C. If humidity in the top of the containers is observed when the content is gelled, it should be dried by any of the well-known methods before the inoculation of the samples. The microscopic organisms, or the samples that contain them, are inoculated by any of the streaking methods, or by any spread surface methods. The inoculated recipients are incubated to temperature from 30 to 45 °C for at least 12 hours.

The evaluation of the results is carried out observing the color and morphology of the isolated colonies in the surface. The observation of blue-greenish colonies with darker center, on an orange bottom of the medium, regular borders and size of 1 to 5 mm, in dependence of the time of incubation, is characteristic for E. *coli* and coliforms, except *Citrobacter* and *Klebsiella* genera which are observed similar, but with violet color on rosy to orange bottom of the medium.

The *Salmonella* non-*typhi* species are observed with a red color center, on a rosy bottom of the medium, regular borders, and 1 to 6 mm diameter, in dependence of the incubation time. The colonies of *Salmonella typhi* and *Proteus* are observed with a color characteristic of the medium due to their transparency, regular borders and size of 1 to 3 mm, in dependence of the incubation time.

Strains of *Pseudomonas aeruginosa* are observed with an orange color with dark center and a greenish pigmentation starting from 24 hours of incubation and emitting yellow-greenish fluorescence under low ultraviolet light (365 nm). Other Gram-negatives organisms remain colorless in the medium.

### Examples.

### Example No. 1.

400 g of the dehydrated composition in powder with the following composition is prepared:

| **COMPONENT** | **G/ 400 G OF MEDIUM** |
|---|---|
| Pancreatic hydrolysate of beef heart (total nitrogen ≈ 10 %) | 63 |
| Enzymatic hydrolysate of milky proteins (total nitrogen ≈ 12 %) | 38 |
| *Saccharomyces* yeast hydrolysate (total nitrogen ≈ 8%) | 38 |

The components related before were previously sifted.

In the composition, the bile salts were included in quality of inhibitors (16,6 g).

Pre-mixture was prepared with 51 g of 3MgO x 4SiO₂ x H₂O, with 1 g of X-gal and 0,4 g of neutral red. All the ingredients were mixed with agar as gelling agent in quantity of 191 g (gel strength of 560 g/ cm²) and sodium carbonate in quantity of 2 g. When the uniformity was achieved and the pH was adjusted to 7,1, the composition was added into flasks hermetically sealed in 15,7 g quantities.

At the same time the C₃H₈O₂ was added in glass flasks for 5 mL.

The content of each flask with the solid composition was poured in an erlenmeyer that contained a mixture of deionized water, and the flask content of C₃H₈O₂; the mixture was agitated, leaving it swelling for 10 minutes, then it was heated to boil for 3 minutes, cooled down until the temperature of 45 °C and distributed in Petri dishes. When the composition was gelled, it was carried out the evaluation of its characteristics and functionality. The differentiation of the colonies and the growth promotion in comparison with the Tryptone Soy Agar, was evaluated with certified strains according to the Table No. 1.

Table 1 Differentiation and growth promotion at 24 hours.

| **Organism** | **Strain ATCC** | **Characteristics of isolated colonies** | **Experimental medium CFU/mL** | **Tryptone Soy Agar FCU/mL** | **Signif. Diff. P≤0,05** |
|---|---|---|---|---|---|
| *Salmonella typhimurium* | 14028 | Red center, regular borders, ≈ 2-3 mm | 430 ± 42,4 | 430 ± 17,6 | - |
| *Escherichia coli* | 25922 | Blue greenish color, regular borders, ≈ 2 mm | 180 ± 21,2 | 200 ± 10,6 | - |
| *Citrobacter freundii* | 9080 | Violet color, regular borders, ≈ 2 mm | 320 ± 98,9 | 370 ± 3,5 | - |

| | | | | | |
|---|---|---|---|---|---|
| Expenmental: Composition described in this invention | | | | | |
| CFU/ mL: Colony Forming Units for each mL of dilution 10⁻⁶ | | | | | |
| + Exist significant differences for p ≤0,05 | | | | | |
| - Do not exist significant differences for p ≤0,05 | | | | | |

A dilution 10⁻¹ of a strain of *Streptococcus faecalis* ATCC 29212 was inoculated and it was inhibited at 24 hours.

Color diffusion from the colonies to the medium was not observed, which allowed the easy enumeration and differentiation of the colonies. Enumeration and early differentiation of late lactose ferments as *Citrobacter* was achieved. Growth promotion of the assayed species was comparable with a general-purpose medium such as Tryptone Soy Agar, and it was statistically demonstrated. Significant differences between the counts obtained in both media were not achieved. The inhibition of the Gram-positives species was observed still at high concentrations of the inoculum.

Subsequently was proceeded to the evaluation of the new composition in comparison with other media taken as references, these ware:
Brilliant Green Agar (BGA): Prepared at a concentration of 50 g/ L of deionized water, shaking the mixture and boiling until complete dissolution. It was sterilized at 121 °C for 15 minutes, cooled down until 45-50°C and distributed in Petri dishes.
Rambach Agar (RA): The content of a flask (to prepare 1 L) was poured in a flask with 1 L of the mixture of the liquid supplement with deionized water. It was shaken and heated with agitation until boil, cooled down until 45-50 °C and distributed in Petri dishes.
Violet Red Bile Agar (VRBA): Prepared at a concentration of 38,5 g/ L in deionized water, it was shaken and heated with agitation until boiling, cooled down until 45-50 °C and distributed in Petri dishes.

According to Table No. 2, besides the growth of the microorganisms in the medium, was compared the growth promotion at a dilution 10⁻⁶ of each inoculum.

**Table 2 Evaluation of the growth different microorganisms (incubation at 37 °C for 24 h).**

| **Organism** | **Medium** | **Characteristic and color of the isolated colonies** | **Growth promotion** | |
|---|---|---|---|---|
| | | | **CFU/mL** | **Signif. Diff. p≤0,05** |
| *Salmonella enteritidis* ATCC 13076 | VRBA | Pink, regular borders, ≤ 1 mm | 15 ± 7,6 | - |
| | RA | Violet red, regular borders, ≈ 1-2 mm | 100 ± 40,0 | |
| | Experimental | Red center, regular borders ≈ 1-2 mm | 130 ± 74,0 | |
| *Salmonella typhimurium* ATCC 14028 | VRBA | Pink, regular borders, ≤ 1 mm | 50 ± 36,5 | - |
| | RA | Violet red, regular borders, ≈ 1-2 mm | 260 ± 201,5 | |
| | Experimental | Red center, regular borders ≈ 1-2 mm | 220 ± 138,0 | |
| *Salmonella typhi* ATCC 19430 | VRBA | No growth | No growth | + |
| | RA | No growth | No growth | |
| | Experimental | Colorless, regular borders, ≈ 2 mm | 130 ± 28,7 | |
| *Proteus vulgaris* ATCC 13315 | VRBA | No growth | No growth | + |
| | RA | No growth | No growth | |
| | Experimental | Colorless to pink regular borders ≈ 2 mm | 225 ± 28,0 | |

| | | | | |
|---|---|---|---|---|
| Experimental: Composition described in this invention | | | | |
| CFU/ mL: Colony Forming Units for each mL of 10⁻⁶ dilution | | | | |
| + Exist significant differences for p ≤0,05 | | | | |
| - Don't exist significant differences for p ≤0,05 | | | | |

The growth of *Proteus vulgaris* in the medium is observed even at high dilutions, the differentiated growth of *Salmonella* non *typhi* species is demonstrated with intense pink to red color in the center of the colonies, well differentiated from the strain of *Salmonella typhi*. The last one grew without inhibition in the composition, even at high dilutions.

Three strains of the coliform group organisms were also inoculated in the composition, using reference traditional media: Violet Red Bile Agar, and Rambach Agar. The growth and characteristic of the colonies are shown in the Table No. 3.

**Table No. 3 Evaluation of the growth of several microorganisms of the coliform group in the composition.**

| **Organism** | **Medium** | **Characteristic and color of the isolated colonies** | **Growth promotion** | |
|---|---|---|---|---|
| | | | **CFU/mL** | **Signif.diff. p≤0,05** |
| *Enterobacter aerogenes* ATCC 13048 | VRBA | Pink, regular borders ≈ 2 mm | 350 ± 130,5 | - |
| | RA | No growth | No growth | |
| | Experimental | Blue-greenish, regular borders, ≈ 2-3 mm | 60 ± 19 | |
| *Escherichia coli* ATCC 25922 | VRBA | Red with bile precipitate, regular borders, ≈ 3 mm | 395 ± 116 | - |
| | RA | Dark blue, with blue halo in the medium, regular borders, ≈ 2 mm | 135 ± 115 | |
| | Experimental | Blue-greenish, regular borders, ≈ 2-3 mm | 270 ± 43 | |
| *Citrobacter freundii* ATCC 9080 | VRBA | Gray, regular borders, ≈ 2 mm | 765 ± 328 | - |
| | RA | Red maroon, regular borders, ≈ 1-2 mm | 305 ± 20 | |
| | Experimental | Pink-violet intense, regular borders, ≈ 2 mm | 460 ± 7,5 | |

| | | | | |
|---|---|---|---|---|
| Experimental: Composition described in the present invention | | | | |
| CFU/ mL: Colony Forming Units for each mL of 10⁻⁶ dilution | | | | |
| + Exist significant differences for p ≤0,05 | | | | |
| - Do not exist significant differences for p ≤0,05 | | | | |

The growth promotion of the assayed coliform microorganisms was statistically similar to that obtained in traditional medium (Violet Red Bile Agar) and higher than the obtained in the Rambach Agar for *Enterobacter aerogenes.* A differentiation of this coliform group was achieved in the experimental composition by their characteristic blue-greenish color, differing from the *Citrobacter* species by their violet color. The differentiation of this late fermenter organism is not achieved in traditional media based on the lactose fermentation.

The composition was evaluated with certified strains, and the incubation time was set for 18 hours, according to the data showed in the Table No. 4.

**Table No. 4. Characteristic of the growth and the colonies at 18 hours**

| **Organism** | **Strain** | **Characteristic of the colonies** |
|---|---|---|
| *Shigella flexneri* | ATCC 12022 | Transparent, regular borders, less than 1 mm |
| *Salmonella typhi* | ATCC 19430 | Transparent, regular borders, brilliants |
| *Salmonella typhimurium* | ATCC 14028 | Red centers, regular borders |
| *Proteus vulgaris* | ATCC 13315 | Transparent, regular borders |
| *Staphylococcus aureus* | ATCC 25923 | Inhibited |
| *Escherichia coli* | ATCC 25922 | Blue greenish, regular borders, brilliants |
| *Pseudomonas aeruginosa* | ATCC 27853 | Clear oranges, darker center, regular borders, greasy aspect |
| *Citrobacter diversus* | sp. | Violets, regular and transparent borders, darker center |
| *Klebsiella pneumoniae* | ATCC 13883 | Violets, regular borders |
| *Enterobacter aerogenes* | ATCC 13048 | Blue-greenish, regular borders, different from *E. coli* (more clears) |

A good differentiation without enrichment at 18 hours was achieved. The strain of *Pseudomonas aeruginosa* was correctly identified, as well as the strains of *Klebsiella* and *Citrobacter.* On the other hand, *Staphylococcus aureus* was totally inhibited in the medium.

The composition was also evaluated in chicken samples contaminated with *Salmonella.* The sample was cut in pieces (360 g) and putted in a polyethylene bag and buffered peptone water was added. The content was agitated, and the inoculum was taken. Serial dilutions were prepared from 10^{- 1} until 10^{- 7}. Two dishes were inoculated, the first one with the more concentrated sample and the second with the minor concentration. Both dishes were incubated for 24 hours at 37 °C. The results are shown in Table No. 5.

**Table No. 5. Characteristic of the colonies growth at 24 hours of incubation.**

| **Characteristic of the observed colonies** | **Organisms identified by the new composition** |
|---|---|
| Red colonies, regular borders, darker center, ≈ 2 mm | *Salmonella* sp. |
| Red colonies, irregular borders, wide and clear halo | *Salmonella sp.* |

At 24 hours, there is a good identification of *Salmonella* without the pre-enrichment or enrichment stages.

An evaluation in 20 samples of *Salmonella* contaminated foods was carried out. A comparison of several combinations of enrichment media and traditional solid media for the identification and isolation of *Salmonella* was carried out, with direct inoculation of the sample in the composition described in this invention and in a parallel experiment, with this proper composition in combination with several enrichment broths. The results are described in Table No. 6.

The media used in the experiment were:
Tetrationate Broth: Prepared by dissolving 46 g of the powder in 1L of deionized water, shaking the mixture until the complete homogenization, and adding a solution of iodine, prepared especially for this medium. It was heated to boiling during 1 minute and distributed into tubes in quantities of 10 mL.
RAMBACH Medium: The content of a flask (to prepare 1 L), was poured in 1 L of a mixture of liquid supplement with deionized water. It was agitated and heated with agitation until boiling, cooled down until 45-50 °C and was distributed in Petri dishes.
Hektoen Enteric Agar: Prepared at a concentration of 75,7 g/L of deionized water. It was shaken and heated with agitation until boiling (1-3 minutes), cooled down until 45-50 °C and distributed in Petri dishes.
Rappaport Vassiliadis Broth: Prepared at a concentration of 30 g/L of deionized water. It was agitated and heated with agitation until boiling (1-3 minutes), was sterilized by autoclaving at 121 °C for 15 minutes, cooled down until 45-50 °C and distributed into tubes.

**Table No. 6. Comparison between the experimental and the traditional media and methods for isolation and identification of Salmonella**

| **Methodology** | **False positive results** |
|---|---|
| Traditional (TT/HE) | 11 |
| Traditional (TT/RAM) | 9 |
| TT/ Experimental | 7 |
| Traditional (RV/HE) | 4 |
| Traditional (RV/RAM) | 4 |
| RV/ Experimental | 2 |
| Experimental (without enrichment) | 0 |

| | |
|---|---|
| Experimental: Composition described in the present invention | |
| HE: Hektoen Enteric Agar | |
| RV: Rappaport Vassiliadis Broth | |
| RAM: Rambach Medium | |
| TT: Tetrationate Broth | |

The number of false positive results in all cases was significantly less in the composition and method described in this invention than in the case of traditional media and methods. The best results were obtained in the case of the direct inoculation of the samples on the composition. With this composition, no false positives where obtained.

### Example No. 2.

The composition was prepared weighting the ingredients separately directly to an erlenmeyer according to the example 1. The sulfured amino acid L-cystine also was added to the composition in quantity of 0,2 g/ L.

A mixture of solid ingredients of the composition was mixed with a mixture of deionized water and C₃H₈O₂. The further preparation, including the solidification (gelling) was executed as described in the example 1.

Certified strains of *Salmonella typhimurium* (ATCC 14028), *Escherichia coli* (ATCC 25922), *Citrobacter freundii* (ATCC 9080) and *Streptococcus faecalis* (ATCC 29212) were inoculated in the composition by streaking in the surface of the gel, until achieve isolated colonies.

A red intense color in the colonies of *Salmonella* at 24 hours was observed and thus made easy the differentiation of this organism even at 21 hours of incubation. The strain of *E. coli* showed blue-green color, different from the other inoculated coliform *(Citrobacter freundii),* which was observed with characteristic violet color colonies. It was evident the inhibition of the strain of *Streptococcus faecalis,* who didn't show growth in any of the tests, even in the case of streaking with concentrated inoculum in 10⁻¹ dilution.

### Example No. 3.

The composition was prepared with the ingredients described in the example 1, with the difference that the heart hydrolysate used was obtained by a papainic hydrolysis (total nitrogen ≈ 12 %). The concentration of this ingredient was similar to the example 1. Another difference consist on the use sodium deoxycholate as inhibitor at a concentration of 1 g/L, and the chromogenic substrate used was x-gal, added in 33 % less concentration than that described in the example 1. These ingredients were weighed in an erlenmeyer flask.

A mixture of deionized water and C₃H₈O₂ was added to the mixture of the previously described solid ingredients and further the composition was prepared according to the method showed in the example 1.

Certified strains of *Escherichia coli* (ATCC 25922), *Salmonella typhimurium* (ATCC 14028), *Proteus vulgaris* (ATCC 13315), *Salmonella enteritidis* (ATCC 13076), *Enterobacter aerogenes* (ATCC 13048), *Salmonella typhi* (ATCC 19430) and

*Proteus mirabilis* (ATCC 7002) were inoculated by streaking in the surface to obtain isolated colonies. The results showed in the Table No. 7 evidence the correctly differentiation of the inoculated species at 18 hours of incubation.

**Table No. 7. Characteristics of the medium and the colonies at 18 hours of incubation.**

| **Organism** | **Color of the medium** | **Color and characteristic of the isolated colonies** |
|---|---|---|
| *Escherichia coli* | Orange | Blue, regular borders, convex, size ≈ 2 mm |
| *Salmonella typhimurium* | Orange-reddish | Red center, regular borders, convex, size ≈ 1,5 - 2 mm |
| *Proteus vulgaris* | Orange | Colorless, regular borders, size ≈ 1,5 mm |
| *Salmonella enteritidis* | Orange-reddish | Red center, regular borders, convex, size ≈ 1,3 mm |
| *Enterobacter aerogenes* | Orange | Clear blue, regular borders, convex, size ≈ 2 mm |
| *Salmonella typhi* | Orange | Colorless, regular borders, convex, size ≈ 1,5 mm |
| *Proteus mirabilis* | Orange | Colorless, regular borders, size ≈ 1,5 mm |

An early and differentiated growth of the species of *Salmonella* non-*typhi* was observed due to the rosy intense color. The strain of *Salmonella typhi* was colorless in the medium and it was smaller in size than the rest of *Salmonella.* The growth of the *Proteus* species was observed, evidencing that this organism is not inhibited, which is a characteristic that differentiates this composition from the rest of the majority of the media distributed already in the market for the differentiation of *Salmonella.*

### Example No. 4.

The composition was prepared according to the example 3, but with the addition of dehydrated egg yolk in quantities of 4 g/ L (V1) and 8 g/ L (V2). The method of preparation was similar to that described in the example 3.

Certified strains of *E*. *coli* (ATCC 25922), *Salmonella typhi* (ATCC 19430), *Proteus vulgaris* (ATCC 13315) and *Streptococcus faecalis* (ATCC 29212) were inoculated by streaking in the gel surface. The observation of the growth was carried out after 24 hours of incubation, and the results at 24 hours are described in the Table No. 8.

**Table No. 8. Functionality of the composition with egg yolk**

| **Organism** | **Variant** | **Color of the medium** | **Color and morphology of the isolated colonies** |
|---|---|---|---|
| *Escherichia coli* | V1 | Orange, opaque | Blue greenish, regular borders, convex, size ≈ 2 mm |
| | V2 | Orange, opaque | Blue greenish, regular borders, convex, size ≈ 2 mm |
| *Salmonella typhimurium* | V1 | Orange-yellowish, opaque | Red center, regular borders, convex, size ≈ 1-2 mm |
| | V2 | Orange-yellowish, opaque | Red center, regular borders, convex, size ≈ 1-2 mm |
| *Klebsiella pneumoniae* | V1 | Rose-orange, opaque | Violet, regular borders, convex, size ≈1-2 mm |
| | V2 | Rose-orange, opaque | Violet, regular borders, convex, size ≈1-2 mm |
| *Salmonella typhi* | V1 | Orange, opaque | Colorless to pinks, regular borders, convex, size ≈ 1 mm |
| | V2 | Orange, opaque | Colorless to pinks, regular borders, convex, size ≈ 1 mm |
| *Proteus vulgaris* | V1 | Orange, opaque | Colorless, regular borders, convex, size ≈ 1 mm |
| | V2 | Orange, opaque | Colorless, regular borders, convex, size ≈ 1 mm |
| *Streptococcus faecalis* | V1 | Orange, opaque | Small colonies, very inhibited, size ≈ 1 mm |
| | V2 | Orange, opaque | Small colonies, very inhibited, size ≈ 1 mm |

The composition according to this invention showed a great opacity in the two variants, and thus facilitated a good contrast for the observation of the microorganism's growth in the gel surface. A differentiation of *Klebsiella pneumoniae* from other coliforms was also observed due to the violet color of the first, thanks to the degradation of the chromogenic substrate and the fermentation of C₃H₈O₂, in presence of a pH indicator. A growth of the *Proteus* species was also observed.

The *Salmonella* species were differentiated also at 24 hours of incubation in the experimental composition, while the growth of *Streptococcus faecalis* was imperceptible at 24 hours.

### Example No. 5.

The composition was prepared according to that described in the example 3, with the further addition of magnesium chloride in quantity of 5 g/L. The method of preparation was similar to the example 3.

Certified strains of *E. coli* (ATCC 25922), *Salmonella typhimurium* (ATCC 14028), *Salmonella typhi* (ATCC 19430), *Salmonella enteritidis* (ATCC 13076), *Streptococcus faecalis* (ATCC 19433), were inoculated by streaking in the gel surface. The observation of the results was executed at 24 hours of incubation and is described in the Table No. 9.

**Table No. 9. Characteristics of the medium and the colonies.**

| **Organism** | **Color of the medium** | **Color and characteristic of the isolated colonies** |
|---|---|---|
| *Escherichia coli* | Orange | Blue greenish, regular borders, convexes, size ≈ 2 mm |
| *Salmonella typhimurium* | Orange-reddish | Red center, regular borders, convex, size ≈ 2 mm |
| *Salmonella enteritidis* | Orange-reddish | Red center, regular borders, convex, size ≈ 2 mm |
| *Salmonella typhi* | Orange | Colorless, regular borders, convexes, size ≈ 1,5 mm |
| *Streptococcus faecalis* | No growth | No growth |

A good growth and an early differentiation of *Salmonella* non-*typhi* species were observed due to their red center. The strain of *E. coli* resulted, as expected, showing blue color of the isolated colonies. The strain of *Streptococcus faecalis* was inhibited in the composition, evidencing their inhibitory power for the Gram-positives organisms.

### Example No. 6.

The composition was prepared with the addition of the nitrogen compound creatinine, in quantity of 0,5 g/ L. The ingredients were weighed in an erlenmeyer.

A mixture of deionized water with C₃H₈O₂ was added to the mixture of solid ingredients and prepared according to the example 1.

A certified strain of *Salmonella typhimurium* (ATCC 14028) was evaluated. It was inoculated by streaking on the gel surface, until obtaining isolated colonies.

An abundant growth of organisms with red color center was achieved at 24 hours of incubation, and at 36 hours changed to dark red, due to the addition of creatinine in the composition.

### Example No. 7.

The evaluation of the composition was carried out with the formulation described in the example 3, evaluating samples of contaminated foods. The detection of *Salmonella* in contaminated meat was carried out in parallel by the traditional method and using the new composition, without pre-enrichment.

Dilutions of the samples were inoculated directly in the surface of the composition by streaking, to obtain isolated colonies. The presence of *Salmonella,* coliforms and other enteric bacteria was detected in 24 hours. For the traditional method, the sample was incubated in peptone buffered solution (24 hours to 37 °C), it was enriched in Tetrationate Broth (24 hours at 43°C) and subsequently it was inoculated in Brilliant Green Agar (prepared according to example 1). The presumptive result for the detection of the presence of *Salmonella* by the traditional method was observed only after three to four days, due to this set of steps.

The suspicious colonies obtained by both methods were picked up and were submitted to the corresponding biochemical tests. The colonies were inoculated in 3 tubes in the media for biochemical analysis (Kligler Iron Agar, Urea Agar, Tryptone Broth and for Indol test in Lysine Iron Agar). The presence of *Salmonella* by both methods was confirmed, achieving a reduction of the total time of the analysis with the new composition of, at least, three days.

## Claims

1. Composition for the detection and early differentiated count of Gram-negative microscopic organisms, wherein it contains
- 0.8 to 4 g/L of inhibitors for Gram-positive microscopic organisms,
- a mixture of substances of protein origin with a total nitrogen content from 9 to 20 %, being in the mixture in a relationship between 2:1 to 24:1 in respect to the content of the inhibitors for Gram-positive organisms, and also containing
- a mixture of organic and inorganic substances, being in this mixture in a relationship between 0.5:1 to 2:1 to the mixture of substances of protein origin, wherein said mixture of organic and inorganic substances comprises oxides of bivalent metals and siliceous compounds, pH indicators, alcohols that can be metabolized by enzymes of at least one of the organisms to identify, chromogenic compounds that can be split by enzymes of at least one of the organisms to identify, and growth promoting substances for Gram-negative organisms.

2. Composition according to claim 1, wherein the mixture of substances of protein origin contains pancreatic or papainic beef heart hydrolysate, enzymatic hydrolysate of milk proteins, microbial origin autolysates or hydrolysates and mixture of egg yolk proteins.

3. Composition according to claim 2, wherein the mixture of the substances of protein origin contains the pancreatic or papainic beef heart hydrolysate at a concentration between 25 and 75 % concerning the mass of said mixture of substances of protein origin.

4. Composition according to claim 2, wherein the mixture of the substances of protein origin contains the enzymatic hydrolysate of milk proteins at a concentration up to 15 % concerning the mass of said mixture of substances of protein origin.

5. Composition according to claim 2, wherein the mixture of the substances of protein origin contains the microbial origin autolysate or hydrolysate at a concentration between 15 and 25 % concerning the mass of said mixture of substances of protein origin.

6. Composition according to claim 2, wherein the mixture of the substances of protein origin contains the egg yolk proteins at a concentration up to 45 % concerning the mass of said mixture of substances of protein origin.

7. Composition according to claim 1, wherein the growth inhibitory substances for Gram-positive organisms are preferably cholic and deoxycholic acids and bile salts.

8. Composition according to claim 1 wherein, within the mixture of organic and inorganic substances, the oxides of bivalent metals and siliceous compounds are preferably 3MgO4SiO₂ H₂O and SiO₂ H₂O, which are used at a concentration from 6 to 32 % respecting to the total mass of the mixture.

9. Composition according to claim 4 wherein, whiting the mixture of organic and inorganic substances, the pH indicators are preferably phenol red and neutral red, which are used at a concentration from 0.03 to 0.18 % respecting to the total mass of the mixtures.

10. Composition according to claim 1 wherein, within the mixture of organic and inorganic substances, the alcohol that can be metabolized by enzymes of at least one of the organisms to identify is preferably C₃H₈O₂, which is used in amounts from 10 to 14 mL/L.

11. Composition according to claim 1 wherein, within the mixture of organic and inorganic compounds, the chromogenic compound that can be split by the action of enzymes of at least on eof the organisms to identify is preferably X-gal, which is used at a concentration from 0,15 to 0.3 % respecting to the total mass of the mixture.

12. Composition according to claim 1 wherein, within the mixture of organic and inorganic compounds, the growth promoting substances for Gram-negative organisms are preferably sodium and magnesium salts, nitrogen compounds of low molecular weight and sulphured amino acids.

13. Composition according to claim 12, wherein the sodium and magnesium salts are preferably magnesium chloride and sodium carbonate, which are at concentrations from 0.03 to 32 % respecting to the total mass of the mixture.

14. Composition according to claim 12, wherein the nitrogen compound of low molecular weights is preferably creatinine, which is used at a concentration up to 3 % respecting to the total mass of the mixture.

15. Composition according to claim 12, wherein the sulphured amino acids are preferably cystine and cysteine, which are used at concentrations up to 1.25 % respecting to the total mass of the mixture.

16. Composition according to claim 1, wherein within the mixture of organic and inorganic substances are included gelling agents, preferably agar with a hardness between 400 and 700 g/cm², which is used at a concentration from 40 to 63 % respecting to the total mass of the mixture.

17. Composition according to claim 1, wherein said composition has a Ph value between 6,8 and 7,4.

18. Method for preparation the composition of claims 1 to 17, wherein said composition is prepared by suspending from 30 to 32 g of the composition in 1 L of a mixture of distilled or deionized water and alcohol, heating and cooling until jellification, inoculating the sample and incubation at temperature from 30 to 45 °C for 12 to 24 hours.

19. Method for the early detection and differentiated count of Gram negative microscopic organisms using a composition according to claims 1-17 as a medium wherein the detection and count of *E. coli* and other coliform organisms is possible by the blue-greenish color of the colonies on the orange bottom of the medium; *Salmonella* non *typhi* for the red color of the center of the colonies on rosy bottom of the medium; *Salmonella typhi* and *Proteus* for the transparency of the colonies; *Citrobacter* and *Klebsiella* for the violet color of the colonies on the pink orange bottom of the medium and *Pseudomonas aeruginosa* for the orange color with darker center of the colony, taking greenish pigmentation starting from 24 hours and producing greenish fluorescence under low ultraviolet light.

## Patentansprüche

1. Zusammensetzung zum Nachweis und zum frühen differenzierten Zählen von gramnegativen mikroskopischen Organismen, welche enthält:
- 0,8 bis 4 g/l Inhibitoren für grampositive mikroskopische Organismen,
- ein Gemisch von von Protein stammenden Substanzen mit einem gesamten Stickstoffgehalt von 9 bis 20 %, das im Gemisch in einem Verhältnis von 2:1 bis 24:1 in bezug auf den Gehalt der Inhibitoren für grampositive Organismen vorliegt, und ebenfalls enthaltend
- ein Gemisch von organischen und anorganischen Substanzen, das in diesem Gemisch in einem Verhältnis von 0,5:1 bis 2:1 bezüglich des Gemischs der von Protein stammenden Substanzen vorliegt, wobei das Gemisch der organischen und anorganischen Substanzen Oxide von zweiwertigen Metallen und siliciumhaltige Verbindungen, pH-Indikatoren, Alkohole, die von Enzymen von zumindest einem der zu identifizierenden Organismen metabolisiert werden können, chromogene Verbindungen, die von Enzymen von zumindest einem der zu identifizierenden Organismen gespalten werden können, und wachstumsfördernde Substanzen für gramnegative Organismen umfaßt.

2. Zusammensetzung nach Anspruch 1,
wobei das Gemisch der von Protein stammenden Substanzen Pankreas- oder Papain-Rinderherz-Hydrolysat, ein enzymatisches Hydrolysat von Milchproteinen, von Mikroben stammende Autolysate oder Hydrolysate und ein Gemisch von Eigelbproteinen enthält.

3. Zusammensetzung nach Anspruch 2,
wobei das Gemisch der von Protein stammenden Substanzen das Pankreas- oder Papain-Rinderherz-Hydrolysat mit einer Konzentration von 25 bis 75 %, auf die Masse dieses Gemischs der von Protein stammenden Substanzen bezogen, enthält.

4. Zusammensetzung nach Anspruch 2,
wobei das Gemisch der von Protein stammenden Substanzen das enzymatische Hydrolysat von Milchproteinen in einer Konzentration von bis zu 15 %, auf die Masse dieses Gemischs der von Protein stammenden Substanzen bezogen, enthält.

5. Zusammensetzung nach Anspruch 2,
wobei das Gemisch der von Protein stammenden Substanzen das von Mikroben stammende Autolysat oder Hydrolysat in einer Konzentration von 15 bis 25 %, auf die Masse dieses Gemischs der von Protein stammenden Substanzen bezogen, enthält.

6. Zusammensetzung nach Anspruch 2,
wobei das Gemisch der von Protein stammenden Substanzen Eigelbproteine in einer Konzentration von bis zu 45 %, auf die Masse dieses Gemischs der von Protein stammenden Substanzen bezogen, enthält.

7. Zusammensetzung nach Anspruch 1,
wobei die wachstumshemmenden Substanzen für grampositive Organismen vorzugsweise Cholinsäure und Desoxycholinsäure und Gallensäuresalze sind.

8. Zusammensetzung nach Anspruch 1,
wobei in dem Gemisch der organischen und anorganischen Substanzen die Oxide von zweiwertigen Metallen und die siliciumhaltigen Verbindungen vorzugsweise 3MgO4SiO₂H₂O und SiO₂ H₂O sind, die in einer Konzentration von 6 bis 32 %, auf die Gesamtmasse des Gemischs bezogen, verwendet werden.

9. Zusammensetzung nach Anspruch 4,
wobei in dem Gemisch der organischen und anorganischen Substanzen die pH-Indikatoren vorzugsweise Phenolrot und Neutralrot sind, die in einer Konzentration von 0,03 bis 0,18 %, auf die Gesamtmasse der Gemische bezogen, verwendet werden.

10. Zusammensetzung nach Anspruch 1,
wobei in dem Gemisch der organischen und anorganischen Substanzen der Alkohol, der von Enzymen von zumindest einem der zu identifizierenden Organismen metabolisiert werden kann, vorzugsweise C₃H₈O₂ ist, der in Mengen von 10 bis 14 ml/l verwendet wird.

11. Zusammensetzung nach Anspruch 1,
wobei in dem Gemisch der organischen und anorganischen Verbindungen die chromogene Verbindung, die durch Wirkung von Enzymen von zumindest einem der zu identifizierenden Organismen gespalten werden kann, vorzugsweise X-Gal ist, das in einer Konzentration von 0,15 bis 0,3 %, auf die Gesamtmasse des Gemischs bezogen, verwendet wird.

12. Zusammensetzung nach Anspruch 1,
wobei in dem Gemisch der organischen und anorganischen Verbindungen die wachstumsfördernden Substanzen für gramnegative Organismen vorzugsweise Natrium- und Magnesiumsalze, Stickstoffverbindungen mit geringem Molekulargewicht und geschwefelte Aminosäuren sind.

13. Zusammensetzung nach Anspruch 12,
wobei die Natrium- und Magnesiumsalze vorzugsweise Magnesiumchlorid und Natriumcarbonat sind, die in einer Konzentration von 0,03 bis 32 %, auf die Gesamtmasse des Gemischs bezogen, vorliegen.

14. Zusammensetzung nach Anspruch 12,
wobei die Stickstoffverbindung mit geringem Molekulargewicht vorzugsweise Kreatinin ist, das in einer Konzentration von bis zu 3 %, auf die Gesamtmasse des Gemischs bezogen, verwendet wird.

15. Zusammensetzung nach Anspruch 12,
wobei die geschwefelten Aminosäuren vorzugsweise Cystin und Cystein sind, die in einer Konzentration von bis zu 1,25 %, auf die Gesamtmasse des Gemischs bezogen, verwendet werden.

16. Zusammensetzung nach Anspruch 1,
wobei im Gemisch der organischen und anorganischen Substanzen gelierende Mittel, vorzugsweise Agar mit einer Härte von 400 bis 700 g/cm², enthalten sind, der in einer Konzentration von 40 bis 63 %, auf die Gesamtmasse des Gemischs bezogen, verwendet wird.

17. Zusammensetzung nach Anspruch 1,
wobei die Zusammensetzung einen pH-Wert von 6,8 bis 7,4 aufweist.

18. Verfahren zur Herstellung der Zusammensetzung nach den Ansprüchen 1 bis 17,
wobei die Zusammensetzung hergestellt wird, indem 30 bis 32 g der Zusammensetzung in 1 Liter eines Gemischs aus destilliertem oder deionisiertem Wasser und Alkohol suspendiert wird, erwärmt und bis zur Gelbildung abgekühlt wird, die Probe eingeimpft wird und 12 bis 24 Stunden bei einer Temperatur von 30 bis 45°C inkubiert wird.

19. Verfahren zum frühen Nachweis und zum differenzierten Zählen von gramnegativen mikroskopischen Organismen unter Verwendung einer Zusammensetzung nach den Ansprüchen 1 bis 1 7 als Medium,
wobei der Nachweis und das Zählen von E. coli und anderen coliformen Organismen wegen der die blau-grünlichen Farbe der Kolonien auf dem orangen Grund des Mediums möglich ist; Salmonella non-typhi wegen der roten Farbe der Mitte der Kolonien auf dem rosaroten Grund des Mediums; Salmonella typhi und Proteus wegen der Transparenz der Kolonien; Citrobacter und Klebsiella wegen der violetten Farbe der Kolonien auf dem rosa-orangen Grund des Mediums und Pseudomonas aeruginosa wegen der orangen Farbe mit einer dunkleren Mitte der Kolonie, wobei die bei 24 Stunden beginnende grünliche Pigmentierung verwendet und unter schwachem ultraviolettem Licht eine grünliche Fluoreszenz erzeugt wird.

## Revendications

1. Composition pour la détection et le dénombrement différencié précoce de microorganismes Gram négatif, qui comprend
- 0,8 à 4 g/litre d'inhibiteurs de microorganismes Gram positif,
- un mélange de substances d'origine protéique dont la teneur totale en azote est comprise entre 9 et 20 %, et qui sont présentes dans le mélange selon un rapport de 2:1 à 24:1 par rapport à la quantité d'inhibiteurs des germes Gram positif, et qui comprend également
- un mélange de substances organiques et inorganiques, qui sont présentes dans ce mélange selon un rapport de 0,5:1 à 2:1 par rapport au mélange de substances d'origine protéique, ledit mélange de substances organiques et inorganiques comprenant des oxydes de métaux divalents et des composés siliceux, des indicateurs de pH, des alcools qui peuvent être métabolisés par les enzymes d'au moins l'un des germes à identifier, des composés chromogènes qui peuvent être dégradés par les enzymes d'au moins l'un des germes à identifier et des substances favorisant la croissance des organismes Gram négatif.

2. Composition selon la revendication 1, dans laquelle le mélange de substances d'origine protéique comprend un hydrolysat pancréatique ou papaïnique de coeur de boeuf, un hydrolysat enzymatique de protéines du lait , des autolysats ou des hydrolysats d'origine microbienne, et des mélanges de protéines de jaune d'oeuf.

3. Composition selon la revendication 2, dans laquelle le mélange des substances d'origine protéique comprend l'hydrolysat pancréatique ou papaïnique de coeur de boeuf à une concentration comprise entre 25 et 75 % en poids dudit mélange de substances d'origine protéique.

4. Composition selon la revendication 2, dans laquelle le mélange des substances d'origine protéique comprend l'hydrolysat enzymatique de protéines du lait à une concentration allant jusqu'à 15 % en poids dudit mélange de substances d'origine protéique.

5. Composition selon la revendication 2, dans laquelle le mélange des substances d'origine protéique comprend l'autolysat ou l'hydrolysat d'origine microbienne à une concentration comprise entre 15 et 25 % en poids dudit mélange de substances d'origine protéique.

6. Composition selon la revendication 2, dans laquelle le mélange des substances d'origine protéique comprend les protéines de jaune d'oeuf à une concentration allant jusqu'à 45 % en poids dudit mélange de substances d'origine protéique.

7. Composition selon la revendication 1, dans laquelle les substances inhibant la croissance des germes Gram positif sont de préférence les acides cholique et désoxycholique et les sels biliaires.

8. Composition selon la revendication 1, dans laquelle les oxydes de métaux divalents et les composés siliceux, au sein du mélange de substances organiques et inorganiques, sont de préférence 3MgO4SiO₂H₂O et SiO₂H₂O, que l'on utilise à une concentration comprise entre 6 et 32 % par rapport au poids total du mélange.

9. Composition selon la revendication 4, dans laquelle les indicateurs de pH au sein du mélange de substances organiques et inorganiques, sont de préférence le rouge de phénol et le rouge neutre, que l'on utilise à une concentration comprise entre 0,03 et 0,18% par rapport au poids total du mélange.

10. Composition selon la revendication 1, dans laquelle l'alcool présent dans le mélange de substances organiques et inorganiques, qui peut être métabolisé par les enzymes d'au moins l'un des germes à identifier, est de préférence le C₃H₈O₂, que l'on utilise dans des quantités comprises entre 10 et 14 ml/litre.

11. Composition selon la revendication 1, dans laquelle le composé chromogène présent au sein du mélange de substances organiques et inorganiques, qui peut être dégradé par l'action des enzymes d'au moins l'un des germes à identifier, est de préférence le X-Gal, que l'on utilise à une concentration comprise entre 0,15 et 0,3 % par rapport au poids total du mélange.

12. Composition selon la revendication 1, dans laquelle les substances favorisant la croissance des germes Gram négatif au sein du mélange de substances organiques et inorganiques sont de préférence des sels de sodium et de magnésium, des composés azotés de faible poids moléculaire et des acides aminés sulfurés.

13. Composition selon la revendication 12, dans laquelle les sels de sodium et de magnésium sont de préférence le chlorure de magnésium et le carbonate de sodium, présents à des concentrations comprises entre 0,03 et 32 % par rapport au poids total du mélange.

14. Composition selon la revendication 12, dans laquelle le composé azoté de faible poids moléculaire est de préférence la créatinine, que l'on utilise à une concentration allant jusqu'à 3 % par rapport au poids total du mélange.

15. Composition selon la revendication 12, dans laquelle les acides aminés sulfurés sont de préférence la cystine et la cystéine, que l'on utilise à une concentration allant jusqu'à 1,25 % par rapport au poids total du mélange.

16. Composition selon la revendication 1, dans laquelle des agents gélifiants sont compris dans le mélange de substances organiques et inorganiques, de préférence de la gélose dont la dureté est comprise entre 400 et 700 g/cm², que l'on utilise à une concentration comprise entre 40 et 63 % par rapport au poids total du mélange.

17. Composition selon la revendication 1, dont la valeur de pH est comprise entre 6,8 et 7,4.

18. Procédé de préparation de la composition selon les revendications 1 à 17, dans lequel ladite composition est préparée en mettant en suspension 30 à 32 g de la composition dans 1 litre d'un mélange composé d'eau distillée ou d'eau désionisée et d'alcool, en chauffant, puis en refroidissant jusqu'à gélification, en inoculant l'échantillon et en incubant à une température comprise entre 30 et 45 °C pendant 12 à 24 heures.

19. Procédé pour la détection précoce et le dénombrement différencié de microorganismes Gram négatif en utilisant une composition selon les revendications 1 à 17 comme milieu, dans lequel la détection et le dénombrement de *E.Coli* et autres germes coliformes est possible par la couleur bleu-vert des colonies sur le fond orange du milieu ; pour *Salmonella* non *typhi* par la couleur rouge au centre des colonies sur le fond rosé du milieu ; pour *Salmonella typhi* et *Proteus* par la transparence des colonies ; pour *Citrobacter* et *Klebsiella* par la couleur violette des colonies sur le fond rose orangé du milieu et pour *Pseudomonas aeruginosa* par la couleur orange plus foncée au centre de la colonie adoptant une pigmentation tirant sur le vert à partir de 24 heures et produisant une fluorescence verdâtre sous une faible lumière ultraviolette.
